(19) Europäisches Patentamt
European Patent Office
Office européen des brevets

(11) EP 2 380 928 B1

# (12) EUROPÄISCHE PATENTSCHRIFT

(45) Veröffentlichungstag und Bekanntmachung des Hinweises auf die Patenterteilung:
**30.05.2018 Patentblatt 2018/22**

(51) Int Cl.:
***C08K 5/3492*** *(2006.01)* ***C07D 251/34*** *(2006.01)*
***C07D 251/32*** *(2006.01)* *C08L 77/00 (2006.01)*

(21) Anmeldenummer: **11163403.6**

(22) Anmeldetag: **21.04.2011**

## (54) TRISALKENYL(ISO)CYANURAT VERNETZER UND VERFAHREN ZUR HERSTELLUNG VERNETZTER ORGANISCHER POLYMERE DAMIT

TRISALKENYL(ISO)CYANURATE CROSS LINKING AGENTS AND PROCESS FOR PRODUCING CROSSLINKED ORGANIC POLYMERS

AGENT DE RÉTICULATION DE TYPE TRISALKENYL(ISO)CYANURATE ET PROCÉDÉ DE FABRICATION DE POLYMÈRES ORGANIQUES RÉTICULÉS À PARTIR DE CELUI-CI

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **22.04.2010 DE 102010028062**

(43) Veröffentlichungstag der Anmeldung:
**26.10.2011 Patentblatt 2011/43**

(73) Patentinhaber: **Evonik Degussa GmbH**
**45128 Essen (DE)**

(72) Erfinder:
- **Schauhoff, Stephanie**
  **63225, Langen (DE)**
- **Trageser, Martin**
  **63571, Gelnhausen-Höchst (DE)**

(56) Entgegenhaltungen:
**EP-A1- 2 530 098 DE-A1- 10 241 914**
**US-A- 3 108 987 US-A1- 2007 208 142**

- **Steven K Henning ET AL: ""Fundamentals of Curing Elastomers with Peroxides and Coagents I: Coagent Structure -Property Relationships" Edited Version of Original Paper Presented at the Spring 167th Technical Meeting of the Rubber Division, American Chemical Society", , 1 January 2005 (2005-01-01), XP055406563, Retrieved from the Internet: URL:http://crayvalley.com.temppublish.com/docs/technical-paper/fundamentals-of-curing-elastomers-with-peroxides-and-coagents-part-1-(1).pdf [retrieved on 2017-09-14]**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 380 928 B1

## Beschreibung

Die Erfindung betrifft ein Verfahren zur Herstellung vernetzter organischer Polymere durch Umsetzen eines Polymers mit dem Vernetzungsmittel Trihexenylisocyanurat sowie neue Verbindungen aus dieser Gruppe.

Stand der Technik:

Die thermischen Anforderungen an Kunststoffmaterialien im Hinblick auf Dauergebrauchstemperatur sowie auf kurzzeitige hohe thermische Belastungen steigen ständig an. Der Hintergrund für steigende Dauergebrauchstemperaturen im Automobilbereich ist z.B. die Entwicklung immer leistungsfähigerer Motoren sowie die immer bessere Schallisolierung, die die Temperatur im Motorraum kontinuierlich ansteigen lässt. Inzwischen werden von den Automobilherstellern Dauergebrauchstemperaturen von bis zu 250°C gefordert. Daher werden mehr und mehr der Standardkautschukmaterialien durch solche mit höherer thermischer Stabilität oder durch hochschmelzende thermoplastische Kunststoffe ersetzt.

Für Anwendungen im Elektronikbereich wie z.B. "connectors", "contact holders" oder Leiterplatten werden Materialien benötigt, die kurzzeitig sehr hohen Temperaturen, die u.U. über ihrem Schmelzpunkt liegen, wie sie z.B. beim Löten von metallischen Verbindungen oder bei Überspannungen auftreten können, widerstehen müssen, ohne ihre Form zu verändern.

Um diesen Anforderungen zu genügen werden Thermoplaste eingesetzt, wobei deren thermische Stabilität durch radikalische Vernetzung weiter erhöht wird. Die Vernetzung kann grundsätzlich durch Coagenzien wie z.B. Triallylcyanurat (TAC) oder Triallylisocyanurat (TAICROS®) deutlich verbessert werden.

Die für diese hohen Anforderungen in Frage kommenden Kunststoffmaterialien (Engineering Polymers und High Performance Polymers) wie z.B. Polyamide der Typen PA 12, PA 11, PA 6, PA 66, PA 46, oder Polyester, haben jedoch so hohe Schmelz- und Verarbeitungstemperaturen (> 180°C), dass z. B. TAC nicht mehr einsetzbar ist, da es bei diesen Temperaturen bereits thermisch polymerisiert. TAICROS kann je nach Verarbeitungszeiten bis max. 250°C eingesetzt werden, da es thermisch stabiler ist. Es hat jedoch den Nachteil, dass es bei diesen Verarbeitungstemperaturen einen relativ hohen Dampfdruck aufweist, was zu einem Verlust an Vernetzer und vor allem zu Emissionsproblemen führt. Es ist dadurch sehr schwer eine gleichmäßige Konzentration an Vernetzer im Compound sicherzustellen.

DE10241914 offenbart die Vernetzung von Fluorkautschuk mit Trimethallylisocyanurat und Dicumylperoxide.

US2007/0208142 offenbart ein Verfahren zur Herstellung eines vernetzten organischen Polymers durch Umsetzung eines Fluorkautschuk mit Trilallylisocyanurat.

US3108987 offenbart ein Verfahren zur Herstellung eines vernetzten organischen Polymers durch Umsetzung eines Polymers mit Trimethylallylcyanurat und Triethylallylcyanurat.

In 2009 wurde von Nippon Kasei ein Patent veröffentlicht, das neue modifizierte Vernetzer der Struktur

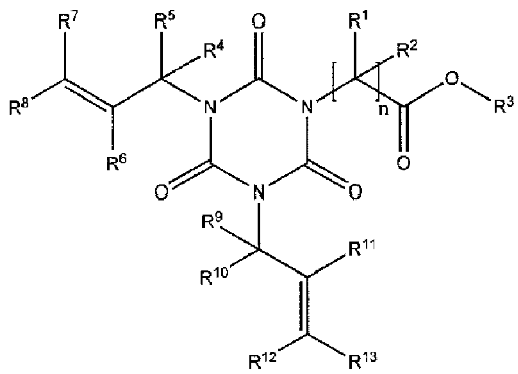

für Elastomere und Thermoplaste bereitstellt, die den Vorteil haben, dass sie Feststoffe sind und sich daher auf einer Walze oder im Extruder leichter einarbeiten lassen, sie eine bessere Verträglichkeit insbesondere mit Fluorkautschken aufweisen und daher zu einer Verringerung des Problems der Formverschmutzung führen.

Diese Verbindungen haben jedoch den Nachteil, dass sie bzgl. der radikalischen Vernetzung nur noch difunktionell und dementsprechend weniger vernetzungseffizient sind. Sie haben weiterhin den Nachteil, dass Sie mit einer Estergruppe eine weitere chemisch weniger stabile Funktion ins Material bringen, die hydrolysieren und niedermolekulare Verbindungen freisetzen kann, was für die chemische Beständikeit der vernetzten Polymere nachteilig ist. Über die thermische Beständigkeit bzw. den Dampfdruck der Verbindung ist nichts bekannt.

Daher besteht Bedarf an einem thermisch stabilen Vernetzer mit einem deutlich niedrigeren Dampfdruck als TAICROS, aber vergleichbarer Vernetzungseffizienz und Polymerisationseigenschaften. Ein solcher Vernetzer ist derzeit

auf dem Markt nicht verfügbar.

Gegenstand der Erfindung ist ein Verfahren zur Herstellung eines vernetzten organischen Polymers durch Umsetzen eines Polymers mit einem Vernetzungsmittel, dadurch gekennzeichnet, dass das Vernetzungsmittel Trihexenylisocyanurat ist (135:123157 CA, Synthesis and characterization of triallylphenoxytriazine and the properties of its copolymer with bismaleimide, Fang, Qiang; Jiang, Luxia, Journal of Applied Polymer Science (2001), 81(5), 1248-1257).

Das erfindungsgemäße Verfahren ist besonders geeignet für thermoplastische Polymere wie z. B. Polyvinylpolymere, Polyolefine, Polystyrole, Polyacrylate, Polymethacrylate, Polyester, Polyamide, Polycarbonate, Polyphenylenether, Polyphenylensulfide, Polyacetale, Polyphenylensulfone, Fluorpolymere oder deren Mischungen, soweit sie als miteinander verträglich bekannt sind.

Bevorzugt sind hochschmelzende Polymere mit Schmelzpunkten > 180°C wie Polystyrole, Polyester, Polyamide, Polycarbonate, Polyphenylenether, Polyphenylensulfide, Polyacetale, Polyphenylensulfone, Fluorpolymere oder deren Mischungen, soweit sie als miteinander verträglich bekannt sind.

Besonders bevorzugt sind Polyamide und Polyester oder deren Mischungen.

Nach dem Stand der Technik werden dann Mischungen aus den geschmolzenen Polymeren und den als Vernetzer wirkenden Verbindungen bei einer Verarbeitungstemperatur hergestellt, die gleich der Schmelztemperatur des Polymeren oder Oligomeren ist oder höher liegt.

Trihexenylisocyanurat wird insbesondere in einer Menge von 0,01 bis 10 Gew.-%, insbesondere 0,5 bis 7 Gew.-%, besonders bevorzugt 1 bis 5 Gew.-%, bezogen auf das vernetzbare Monomer, Oligomer und/oder Polymer eingesetzt.

Die Menge des Vernetzers hängt im Allgemeinen von dem spezifischen Polymer und dem beabsichtigten Einsatzgebiet dieses Polymers ab. Eine Kombination mit anderen vernetzend wirkenden Komponenten wird nicht ausgeschlossen, ist aber nicht notwendig.

Die Vernetzung kann peroxidisch oder durch Elektronenstrahlvernetzung erfolgen. Bei den erfindungsgemäß besonders bevorzugten eingesetzten hochschmelzenden Polymeren ist nur die Elektronenstrahlvernetzung anwendbar, die bei Raumtemperatur erfolgt, während die Verarbeitungstemperatur von Peroxiden maximal 150°C beträgt, und bei peroxidisch vernetzten Systemen die Vernetzungstemperatur bei 160 bis 190 °C liegt. Eine peroxidische Vernetzung erfolgt daher bevorzugt bei Polymeren oder Oligomeren mit einem Schmelzpunkt von 100 bis 150°C.

Die erfindungsgemäß eingesetzten Vernetzer sind jedoch auch für die Vernetzung von radikalisch vernetzbaren Elastomeren wie z.B. Naturkautschuk, Isoprenkautschuk,Butadienkautschuk, Ethylenpropylenkautschuk, Nitrilkautschuk, hydrierter Nitrilkautschuk, Chloroprenkautschuk, Chlorsulfonylpoyethylen, Acrylkautschuk, Ethylenacrylkautschuk, Fluorkautschuk, Ethylen-VinylacetatCopolymere, Silikonkautschuk oder deren Mischungen geeignet, wobei sie insbesondere bei höheren Verarbeitungstemperaturen Vorteile bieten und aufgrund der längeren Seitenketten mit unpolaren Elastomeren wie z.B. Fluorkautschuk besser verträglich sind.

Die Nomenklatur der Kohlenwasserstoffreste entspricht der aus dem Handbook of Chemistry and Physics, 52nd Edition, 1972-1972.

Die Herstellung der eingesetzten Verbindungen erfolgt nach dem folgenden Verfahren:

TAC-Analoge:

Die den Substituenten entsprechenden Alkohole oder OH-Gruppen enthaltenden Verbindungen werden mit etwas Wasser unter Kühlung vorgelegt und dann bei Reaktionstemperaturen von 5 bis 20°C, meist 7 bis 15°C, Cyanurchlorid und Natronlauge im Verlauf von 1 bis 2 Stunden simultan zudosiert. Nach Zugabe erfolgt eine Aufarbeitung und Abtrennung der org. Matrix durch Zugabe von Wasser und entsprechender Phasentrennung.

Die org. Matrix wird dann destillativ von Resten Wasser und von Lösemittel (eingesetzte Alkohole bzw. OH-Gruppen enthaltende Verbindungen) befreit und somit die Zielprodukte erhalten.

Die durch Destillation zurückgewonnenen Alkohole oder OH-Gruppen enthaltenden Verbindungen können dem Prozess wieder zugeführt werden. Die Synthesen erfolgen im Molverhältnis Cyanurchlorid:Alkohol bzw. OH-Gruppen enthaltende Verbindung:Natronlauge von 1,0:3,3:3,1 bis 1,0:6,0:3,5, insbesondere aber bei 1,0:5,1:3,36.

Die Identität der Verbindungen wurde über HPLC-MS bestätigt.

TAICROS-Analoge (entsprechend Trihexenylisocyanurat)

Die Synthesen erfolgen aus Natriumcyanurat (Tri-natriumsalz der Isocyanursäure) und den entsprechenden Alkenchloriden bzw. mit Chlorid substituierten Verbindungen insbesondere in Dimethylformamid als Lösemittel, wobei alle Komponenten, bevorzugt zusammen vorgelegt werden und dann bei 120 bis 145°C für 5 bis 8 Stunden umgesetzt werden. Nach Abkühlung wird vom Salz abfiltriert und die erhaltene org. Phase destillativ unter Vakuum vom Dimethylformamid befreit und somit die Zielprodukte erhalten.

Die Synthesen erfolgten im Molverhältnis 1:3 der Reaktanden Natriumcyanurat und Chloride.

Die Identität der Verbindungen wurde über HPLC-MS bestätigt.

Gegenstand der Erfindung sind ebenfalls vernetzbare Zusammensetzungen, enthaltend ein Polymer, ausgewählt aus der Gruppe

Polyvinylpolymere, Polyolefine, Polystyrole, Polyacrylate, Polymethacrylate, Polyester, Polyamide, Polycarbonate, Polyphenylenether, Polyphenylensulfide, Polyacetale, Polyphenylensulfone, Fluorpolymere oder deren Mischungen, insbesondere Polyamide und Polyester oder deren Mischungen oder Elastomeren ausgewählt aus der Gruppe Naturkautschuk, Isoprenkautschuk,Butadienkautschuk, Ethylenpropylenkautschuk, Nitrilkautschuk, hydrierter Nitrilkautschuk, Chloroprenkautschuk, Chlorsulfonylpoyethylen, Acrylkautschuk, Ethylenacrylkautschuk, Fluorkautschuk, Ethylen-VinylacetatCopolymere, Silikonkautschuk oder deren Mischungen und Trihexenylisocyanurat.

Beispiele

Anwendungstechnische Prüfungen der Vernetzer:
In den Beispielen werden folgende Verbindungen eingesetzt:

| | |
|---|---|
| TAC: | Triallylcyanurat |
| TAIC (TAICROS®): | Triallylisocyanurat |
| TMAC: | Trimethylallylcyanurat |
| TMAIC: | Trimethylallylisocyanurat |
| THC: | Trihexenylcyanurat |
| THIC: | Trihexenylisocyanurat |
| TAPC: | Triallylphenylcyanurat |

1. Eigenschaften der Vernetzer:

1.1 Gewichtsverlust bei Erhitzen:

Wie in der folgenden Tabelle gezeigt, haben die neuen Verbindungen einen deutlich niedrigeren Dampfdruck als TAIC. Dies drückt sich in einem deutlich niedrigeren Gewichtsverlust bei Erhitzen auf höhere Temperaturen aus.

Tabelle 1

| | TAC | TAIC | TMAIC | TMAC | THC | THIC | TAPC |
|---|---|---|---|---|---|---|---|
| MG [g/mol] | 249,27 | 249,27 | 291,35 | 291,35 | 375,51 | 375,51 | 477,55 |
| TGA (Bed.: von RT bis 350°C, Heizrate 10 K/min unter Luft) | | | | | | | |
| Gew.Verlust (%) bei | | | | | | | |
| 200 °C | 1,91 | 3,7 | 2,0 | 0.9 | 1,1 | 4,9 | 0,8 |
| 250°C | 4,64 | 26,1 | 15,1 | 5,4 | 2,6 | 6,4 | 0,9 |
| 300°C | 7,7* | 99,8 | 61,8 | 36,3 | 12,9 | 10,9 | 3,0 |
| 350°C | 48,4* | 99,9 | 69,3* | 65,4 | 36,5 | 24,6 | 16,2 |
| * Material polymerisiert!! | | | | | | | |

<u>1.2 Thermische Stabilität:</u>

Zur Untersuchung der thermischen Stabilität wurden kleine Mengen (50 - 100 mg) der Substanzen bei verschiedenen Temperaturen gelagert und die Änderung des Monomergehaltes über die Zeit mittels HPLC-Analyse verfolgt. Alle Verbindungen waren dabei mit 100 ppm MEHQ (Methylhydrochinon) stabilisiert.

In den folgenden Tabellen sind die Restmonomergehalte in % angegeben:

Tabelle 2

| **160°C** | TAC | TAICROS | TAICROS-M | TMAC | THC | THIC | TAPC |
|---|---|---|---|---|---|---|---|
| | | | | | | | |
| Time **(hours)** | Gehalt [%] | Gehalt [%] | Gehalt [%] | Gehalt [%] | Gehalt [%] | Gehalt [%] | Gehalt [%] |
| 0 | 100 | 100 | 100 | 100,0 | 100,0 | 100.0 | 100,0 |

(fortgesetzt)

| **160°C** | TAC | TAICROS | TAICROS-M | TMAC | THC | THIC | TAPC |
|---|---|---|---|---|---|---|---|
| | | | | | | | |
| Time **(hours)** | Gehalt [%] | Gehalt [%] | Gehalt [%] | Gehalt [%] | Gehalt [%] | Gehalt [%] | Gehalt [%] |
| 1 | 90 | 98 | 100 | 100,0 | 100,0 | 100,0 | 100,0 |
| 5 | 0 | 96,2 | 100 | 100,0 | 100,0 | 100,0 | 100,0 |

Tabelle 3

| **200°C** | TAC | TAICROS | TAICROS-M | TMAC | THC | THIC | TAPC |
|---|---|---|---|---|---|---|---|
| | | | | | | | |
| Time **(min)** | Gehalt [%] | Gehalt [%] | Gehalt [%] | Gehalt [%] | Gehalt [%] | Gehalt [%] | Gehalt [%] |
| 0 | 100,0 | 100,0 | 100,0 | 100,0 | 100 | 100 | 100 |
| 2 | 94,5 | 100,0 | 94,3 | 96,7 | 96,2 | 93,5 | 90,9 |
| 5 | 53,8 | 85,4 | 84,7 | 79,7 | 91,8 | 92,6 | 79,3 |
| 10 | 41,1 | 23,6 | 74,5 | 52,1 | 81,2 | 90,7 | 71,6 |

Tabelle 4

| **225 °C** | TAC | TAICROS | TAICROS-M | TMAC | THC | THIC | TAPC |
|---|---|---|---|---|---|---|---|
| | | | | | | | |
| Time **(min)** | Gehalt [%] | Gehalt [%] | Gehalt [%] | Gehalt [%] | Gehalt [%] | Gehalt [%] | Gehalt [%] |
| 0 | 100,0 | 100,0 | 100,0 | 100,0 | 100 | 100 | 100 |
| 2 | 0 * | 60,8 | 89,2 | 73,1 | 88,2 | 88,5 | 80,3 |
| 5 | 0 * | 24,0 | 73,8 | 47,6 | 75,4 | 76,9 | 66,3 |
| 10 | 0 * | 21,6 | 65,8 | 36,2 | 56,5 | 65 | 49,1 |

Tabelle 5

| **250°C** | TAC | TAICROS | TAICROS-M | TMAC | THC | THIC | TAPC |
|---|---|---|---|---|---|---|---|
| time **(min)** | Gehalt [%] | Gehalt [%] | Gehalt [%] | Gehalt [%] | Gehalt [%] | Gehalt [%] | Gehalt [%] |
| | % | % | | | | | |
| 0 | n.b. | 100,0 | 100,0 | 100 | 100 | 100 | 100 |
| 2 | n.b. | 43,6 | 72,7 | 46,9 | 77,1 | 78,5 | 78,0 |
| 5 | n.b. | 25,4 | 63,7 | 31,2 | 42 | 60,2 | 46,8 |
| 10 | n.b. | 24,3 | 58,5 | 28,8 | 29,9 | 38,1 | 27,2 |
| * Polymer, Restmonomergehalt nicht bestimmt | | | | | | | |

Die neuen Verbindungen können kurzzeitig deutlich höhere Verarbeitungstemperaturen überstehen bzw. zeigen eine deutlich langsamere thermisch induzierte Homopolymerisation.

2. Anwendungsbeispiele:

Polyamid 6 (Ultramid B3K, BASF) wurde mit je 3 Gew% TAICROS® (Evonik), THC bzw. TAPC im Extruder compoundiert.

Analog wurde Polyamid 66 (Ultramid A3K) mit je 3 Gew% THC bzw. TAPC im Extruder gemischt. Die Extrusion mit TAIC war wegen des zu hohen Dampfdruckes und einsetzender Polymerisation mit PA 66 nicht möglich.

Um die Dosierung der Vernetzer zu erleichtern wurden diese in Form eines Masterbatches zudosiert. Die Masterbatches wurden im Falle der flüssigen Vernetzer durch direktes Aufziehen der Flüssigkeiten auf ein poröses Polyamid (Accurell MP 700) und bei den festen Vernetzern durch aufziehen einer Lösung des Vernetzers auf Accurell und anschließende Trocknung hergestellt. Die Konzentration der Masterbatches betrug 30 %, d.h. es wurden 10% PA-Masterbatch (PA MB) zugemischt. Zum Vergleich wurden Polyamidproben mit unbeladenem Accurell MP 700 extrudiert. Bei der Compoundierung wurde bei PA 6 mit TAICROS deutliche "Nebelbildung" (Abdampfen von TAICROS aus dem Polymerstrang) mit entsprechender Geruchsbelästigung am Extruderausgang beobachtet. Dies war mit den beiden neuen Vernetzern THC und TAPC nicht der Fall.

2.1 MFI:

Nach der Extrusion wurde anhand des MFI (melt flow index) untersucht, ob eine "Vorpolymerisation" bei der Compoundierung stattgefunden hat. Durch Extrusion mit unbeladenem Accurell wird der MFI etwas erniedrigt, d.h. die Schmelzeviskosität steigt etwas an.

Bei PA 6 führen TAICROS sowie auch THC im Vergleich dazu zu einer leichten Reduktion des MFI, was auf eine minimale An-Vernetzung schließen lässt. TAICROS M beeinflusst den MFI nicht, während TAPC zu einer deutlichen Erhöhung des MFI, d.h. einer Reduzierung der Schmelzeviskosität führt. Dies beruht vermutlich darauf, daß die Verbindung als Gleitmittel wirkt.

Im PA 66 führen alle getestetetn Vernetzer zu einer leichten Erhöhung des MFI. Offenbar macht sich bei der höheren Bestimmungstemperatur von 280°C gegenüber 250°C die Gleitmittelwirkung stärker bemerkbar. Jedenfalls kann man davon ausgehen, daß bei der Compoundierung keine deutliche vorzeitige Vernetzung stattgefunden hat.

Tabelle 6

| **PA 6** | MFI (250°C/2,16 kg) |
|---|---|
| | g/10 min. |
| PA 6 Ausgangsmaterial | 35,0 |
| PA 6 + PA 6 porous extr. | 31,7 |
| PA 6 + 3% TAICROS (PA MB) | 27,9 |
| PA 6 + 3% TAICROS M (PA MB) | 32,7 |
| PA 6 + 3% THC (PA MB) | 25,3 |
| PA 6 + 3% TAPC (PA MB) | 44,8 |

Tabelle 7

| **PA 66** | MFI (280°C/2,16 kg) |
|---|---|
| | g/10 min. |
| PA 66 Ausgangsmaterial | 52,8 |
| PA 66 + PA 6 porous extr. | 46,3 |
| PA 66 + 3% TAICROS M (PA MB) | 55,8 |
| PA 66 + 3% THC (PA MB) | 56,5 |
| PA 66 + 3% TAPC (PA MB) | 89,2 |

2.2 Vernetzungsgrad:

Granulatproben aller Mischungen wurden dann mit 120 und 200 kGy elektronenstrahlvernetzt. An den Granulatproben wurde anschließend der Vernetzungsgrad über den Gelgehalt wie folgt ermittelt:
Jeweils ca 1,0 g des Granulates wurden eingewogen und mit 100 ml m-Kresol versetzt,
unter Rühren auf auf Siedetemperatur erhitzt und min. 3 Std. unter Rückfluss behandelt. Nach dieser Zeit war das nicht vernetzte Polyamid komplett gelöst. Bei den vernetzten Proben, wurde der unlösliche Anteil abfiltriert und mit Toluol gewaschen. Die Rückstände wurden dann im Vakuumschrank bei 120°C bis zu 7 Std. getrocknet und gewogen. Der unlösliche Anteil entspricht dem Vernetzungsgrad.

Tabelle 8

| **PA 6** | Gelgehalt (120 kGy) |
|---|---|
| | % |
| PA 6 Ausgangsmaterial | 5,85 |
| PA 6 + 3% TAICROS (PA MB) | 100 |
| PA 6 + 3% TAICROS M (PA MB) | 100 |
| PA 6 + 3% THC (PA MB) | 100 |
| PA 6 + 3% TAPC (PA MB) | 100 |

Tabelle 9

| **PA 66** | Gelgehalt (120 kGy) |
|---|---|
| | % |
| PA 66 Ausgangsmaterial | 0, 67 |
| PA 66 + 3% TAICROS M (PA MB) | 100 |
| PA 66 + 3% THC (PA MB) | 100 |
| PA 66 + 3% TAPC (PA MB) | 100 |

Da alle Mischungen schon bei 120 kGy vollständig vernetzt waren, wurden die Gelgehalte der bei 200 kGy vernetzten Proben nicht mehr bestimmt.

2.3 Verschlaufungsdichte:

Eine weitere Information über die Vernetzung liefert die "Verschlaufungsdichte" berechnet aus dem E-Modul nach der Formel

$$E/3 = n \times k \times T$$

mit

n = Verschlaufungsdichte
k = Bolzmann Konstante = 1,38 x 1023 J/K
T = Temperatur in K

Danach ergeben sich folgende Werte: s. Tabelle.
Nach dieser Methode ist der Unterschied zu PA ohne Vernetzer nicht so ausgeprägt, wie beim Gelgehalt, dennoch zeigt sich eine deutliche Erhöhung der Verschlaufungsdichte durch die Vernetzer. Die tendenziell niedrigeren Werte mit den neuen Vernetzern im Vergleich zu TAICROS sind vermutlich auf die höheren Molekulargewichte, d.h. niedrigerer mol-Zahl bei 3% Zusatz, zurückzuführen, wobei TAPC tendenziell effizienter ist als THC.

Tabelle 10

| Bezeichnung | PA 6 extrudiert | PA 6 + 3% TAICROS (PA MB) | PA 6 + 3% TAICROS M (PA MB) | PA 6 + 3% THC (PA MB) | PA 6 + 3% TAPC (PA MB) |
|---|---|---|---|---|---|
| **Bestrahlung** | **no EB** | **keine** | **keine** | **keine** | **keine** |
| Verschlaufungsdichte | 1, 98 | 1,81 | 1,85 | 1,78 | 1,94 |
| **Bestrahlung** | **120 kGy** | **120** | **120** | **120** | **120** |
| Verschlaufungsdichte | 2,06 | 2,20 | 2,15 | 2,07 | 2,12 |
| **Bestrahlung** | **200 kGy** | **200** | **200** | **200** | **200** |
| Verschlaufungsdichte | 2,10 | 2,22 | 2,20 | 2,15 | 2,20 |

Tabelle 11

| Bezeichnung | PA 66 extrudiert | PA 66 + 3% TAICROS M (PA MB) | PA 66 + 3% THC (PA MB) | PA 66 + 3% TAPC (PA MB) |
|---|---|---|---|---|
| **Bestrahlung** | **no EB** | **keine** | **keine** | **keine** |
| Verschlaufungsdichte | 2,08 | 2,14 | 2,02 | 2,12 |
| **Bestrahlung** | **120 kGy** | **120** | **120** | **120** |
| Verschlaufungsdichte | 2,18 | 2,33 | 2,26 | 2,28 |
| **Bestrahlung** | **200 kGy** | **200** | **200** | **200** |
| Verschlaufungsdichte | 2,19 | 2,37 | 2,32 | 2,35 |

2.4 Restvernetzergehalt:

An den vernetzen Granulaten wurde außerdem der Restvernetzergehalt durch Totalextraktion mit Methanol bestimmt. Es zeigte sich, daß schon bei 120 kGy alle Vernetzer >99% umgesetzt werden.

Tabelle 12

| **PA 6** | Restvernetzergehalt nach Bestrahlung mit 120 kGy | Restvernetzergehalt nach Bestrahlung mit 200 kGy |
|---|---|---|
| | % | % |
| PA 6 Ausgangsmaterial | 0,00 | 0,00 |
| PA 6 + 3% TAICROS (PA MB) | 0,00 | 0,00 |
| PA 6 + 3% TAICROS M (PA MB) | 0,05 | 0,00 |
| PA 6 + 3% THC (PA MB) | 0,67 | 0,43 |
| PA 6 + 3% TAPC (PA MB) | 0,02 | 0,00 |

Tabelle 13

| PA 66 | Restvernetzergehalt nach Bestrahlung mit 120 kGy | Restvernetzergehalt nach Bestrahlung mit 200 kGy |
|---|---|---|
| | % | % |
| PA 66 Ausgangsmaterial | 0,00 | 0,00 |
| PA 66 + 3% TAICROS M (PA MB) | 0,30 | 0,00 |
| PA 66 + 3% THC (PA MB) | 0,16 | 0,13 |
| PA 66 + 3% TAPC (PA MB) | 0,00 | 0,00 |

2.5 Kurzzeit-Wärmeformbeständigkeit (Lötkolbentest):

Weiterhin wurde an den Granulaten ein "Lötkolbentest" durchgeführt. Dabei wird ein Metalldorn mit einer hohen Temperatur unter definiertem Druck für einige Sekunden auf den Prüfkörper gedrückt und die Eindringtiefe gemessen. Dieser Test simuliert eine kurzzeitige hohe thermische Belastung für die die hier beschriebenen Materialien besonders geeignet sind.

Tabelle 14

| PA 6 | Bestrahlungsdosis/kGy | | |
|---|---|---|---|
| nach Extrusion | 0 | 120 | 200 |
| PA 6 Ausgangsmaterial | 2,07 | n.b. | n.b. |
| PA 6 + PA 6 porous extr. | 2,37 | 2,37 | 2,26 |
| PA 6 + 3% TAICROS (PA MB) | 2,05 | 0,18 | 0,16 |
| PA 6 + 3% TAICROS M (PA MB) | 2,17 | 0,29 | 0,30 |
| PA 6 + 3% THC (PA MB) | 2,13 | 0,83 | 0,48 |
| PA 6 + 3% TAPC (PA MB) | 2,24 | 2,01 | 1,39 |

Tabelle 15

| PA 66 | Bestrahlungsdosis/kGy | | |
|---|---|---|---|
| | 0 | 120 | 200 |
| PA 66 Ausgangsmaterial | 1,57 | n.b. | n.b. |
| PA 66 + PA 6 porous extr. | 1,72 | 1, 61 | 1,63 |
| PA 66 + 3% TAICROS M (PA MB) | 1,79 | 0,23 | 0,21 |
| PA 66 + 3% THC (PA MB) | 1,77 | 0,49 | 0,30 |
| PA 66 + 3% TAPC (PA MB) | 1,76 | 1,16 | 1,27 |

Die Ergebnisse bestätigen, dass durch den Zusatz der Vernetzer die Vernetzung des Polyamids erheblich erhöht und dadurch eine hohe thermomechanische Stabilität/Wärmeformbeständigkeit bei Kurzzeitbelastung erreicht wird. Die Unterschiede zwischen den Vernetzern ergeben sich zum größten Teil aus den unterschiedlichen Molekulargewichten, wobei TAPC bzgl. dieser Eigenschaft tendenziell etwas schlechter zu sein scheint als THC.

Um sicherzustellen, daß auch die übrigen mechanischen Eigenschaften der Materialien den Anforderungen entsprechen, wurden aus den Compounds Prüfkörper hergestellt, unter den gleichen Bedingungen wie oben elektronenstrahl-vernetzt und die mechanischen Eigenschaften im Zugversuch sowie die Wärmeformbeständigkeit (HDT) bestimmt.

2.6 Langzeit-Wärmeformbeständigkeit (HDT):

Wie TAICROS bzw. TAICROS M verbessern die neuen Vernetzer die Wärmeformbeständigkeit (HDT=heat distortion temperature) des Polyamids. Die tendenziell niedrigeren Werte mit den neuen Vernetzern sind vermutlich wie oben schon erwähnt auf eine niedrigere Anzahl von Vernetzunsgstellen aufgrund des höheren Molekulargewichtes, d.h. niedrigerer mol-Zahl bei 3% Zusatz, zurückzuführen.

Tabelle 16

| PA 6 | HDT | Bestrahlungsdosis/kGy | | |
|---|---|---|---|---|
| | **Methode** | **0** | **120** | **200** |
| PA 6 ohne Vernetzer | A | 51 | 54 | 55 |
| PA 6 + 3% TAICROS (PA MB) | A | 49 | 63 | 69 |
| PA 6 + 3% TAICROS M (PA MB) | A | 50 | 64 | 64 |
| PA 6 + 3% THC (PA MB) | A | 49 | 60 | 60 |
| PA 6 + 3% TAPC (PA MB) | A | 50 | 61 | 60 |
| | **Methode** | | | |
| PA 6 Ausgangsmaterial | B | 183 | 180 | 180 |
| PA 6 + 3% TAICROS (PA MB) | B | n.b. | 179 | 183 |
| PA 6 + 3% TAICROS M (PA MB) | B | 183 | 187 | 184 |
| PA 6 + 3% THC (PA MB) | B | 159 | 183 | 187 |
| PA 6 + 3% TAPC (PA MB) | B | 169 | 180 | 183 |

Tabelle 17

| **PA 66** | **HDT** | **Bestrahlungsdosis/kGy** | | |
|---|---|---|---|---|
| | **Methode** | **0** | **120** | **200** |
| PA 66 Ausgangsmaterial | A | 60 | 67 | 66 |
| PA 66 + 3% TAICROS M (PA MB) | A | 61 | 77 | 77 |
| PA 66 + 3% THC (PA MB) | A | 58 | 82 | 76 |
| PA 66 + 3% TAPC (PA MB) | A | 56 | 69 | 74 |
| | Methode | | | |
| PA 66 Ausgangsmaterial | B | 207 | 210 | 213 |
| PA 66 + 3% TAICROS M (PA MB) | B | 220 | 223 | 225 |
| PA 66 + 3% THC (PA MB) | B | 208 | 223 | 222 |
| PA 66 + 3% TAPC (PA MB) | B | 216 | 216 | 216 |

2.7 Mechanische Eigenschaften:

Bei den mechanischen Eigenschaften zeigt TAPC ein spröderes Verhalten als THC.

**Zugversuche nach ISO 527 von PA 6 und 6.6 Schulterstäben**

| Bezeichnung | | PA 6 extrudiert | PA 6 + 3% TAICROS (PA MB) | PA 6 + 3% TAICROS M (PA MB) | PA 6 + 3% THC (PA MB) | PA 6 + 3% TAPC (PA MB) |
|---|---|---|---|---|---|---|
| **Bestrahlung** | | **no EB** | **keine** | **keine** | **keine** | **keine** |
| Elastizitätsmodul Et | MPA | 2396 | 2201 | 2242 | 2157 | 2358 |
| Zugfestigkeit $Q_M$ | MPa | 61,0 | 58,9 | 59,9 | 57,2 | 56,2 |
| Bruchdehnung $\varepsilon_B$ | % | 75 | 105 | 61 | 83 | 32 |
| | | | | | | |
| **Bestrahlung** | **kGy** | **120 kGy** | **120** | **120** | **120** | **120** |
| Elastizitätsmodul Et | MPA | 2496 | 2666 | 2608 | 2510 | 2568 |
| Zugfestigkeit $Q_M$ | MPa | 64,8 | 71,3 | 71,7 | 67,1 | 69,7 |
| Bruchdehnung $\varepsilon_B$ | % | 56 | 47 | 33 | 92 | 35 |
| | | | | | | |
| **Bestrahlung** | **kGy** | **200 kGy** | **200** | **200** | **200** | **200** |
| Elastizitätsmodul Et | MPA | 2542 | 2698 | 2666 | 2606 | 2674 |
| Zugfestigkeit $Q_M$ | MPa | 65,6 | 73,1 | 72,5 | 68,4 | 70,9 |
| Bruchdehnung $\varepsilon_B$ | % | 61 | 40 | 36 | 56 | 38 |

Tabelle 1

| Bezeichnung | | PA 66 extrudiert | PA 66 + 3 % TAICROS M (PA MB) | PA 66 + 3% THC (PA MB) | PA 66 + 3% TAPC (PA MB) |
|---|---|---|---|---|---|
| **Bestrahlung** | | **keine** | **keine** | **keine** | **keine** |
| Elastizitätsmodul Et | MPA | 2519 | 2598 | 2450 | 2572 |
| Zugfestigkeit $Q_M$ | MPa | 68,4 | 70,1 | 68,5 | 70,0 |
| Bruchdehnung $\varepsilon_B$ | % | 69 | 42 | 43 | 48 |
| | | | | | |
| **Bestrahlung** | **kGy** | **120** | **120** | **120** | **120** |
| Elastizitätsmodul Et | MPA | 2640 | 2825 | 2743 | 2760 |
| Zugfestigkeit $Q_M$ | MPa | 70,7 | 77,6 | 76,4 | 75,2 |
| Bruchdehnung $\varepsilon_B$ | % | 54 | 31 | 40 | 35 |
| | | | | | |
| **Bestrahlung** | **kGy** | **200** | **200** | **200** | **200** |
| Elastizitätsmodul Et | MPA | 2662 | 2880 | 2818 | 2847 |
| Zugfestigkeit $Q_M$ | MPa | 71,1 | 78,4 | 77,0 | 76,3 |
| Bruchdehnung $\varepsilon_B$ | % | 43 | 29 | 45 | 39 |

## Patentansprüche

1. Verfahren zur Herstellung eines vernetzten organischen Polymers durch Umsetzung eines Polymers mit einem Vernetzungsmittel, **dadurch gekennzeichnet, dass** das Vernetzungsmittel Trihexenylisocyanurat ist.

2. Verfahren gemäß Anspruch 1 , **dadurch gekennzeichnet, dass** als Polymere thermoplastische Polymere, ausgewählt aus der Gruppe, enthaltend Polyvinylpolymere, Polyolefine, Polystyrole, Polyacrylate, Polymethacrylate, Polyester, Polyamide, Polycarbonate, Polyphenylenether, Polyphenylensulfide, Polyacetale, Polyphenylensulfone, Fluorpolymere oder deren Mischungen, soweit sie als miteinander verträglich bekannt sind, eingesetzt werden.

3. Verfahren gemäß den Ansprüchen 1 bis 2, **dadurch gekennzeichnet, dass** die Menge an Trihexenylisocyanurat von 0,01 bis 10 Gew.-%, bezogen auf das vernetzbare Polymer beträgt.

4. Verfahren gemäß den Ansprüchen 1 bis 3, **dadurch gekennzeichnet, dass** man die Vernetzung in Abhängigkeit von dem zu vernetzenden Polymers entweder unter Zusatz eines geeigneten Peroxids bei einer Temperatur von 160 bis 190°C durchführt oder durch Elektronenstrahlvernetzung bei Raumtemperatur.

5. Vernetzbare Zusammensetzung, enthaltend ein Polymer, ausgewählt aus der Gruppe Polyvinylpolymere, Polyolefine, Polystyrole, Polyacrylate, Polymethacrylate, Polyester, Polyamide, Polycarbonate, Polyphenylenether, Polyphenylensulfide, Polyacetale, Polyphenylensulfone, Fluorpolymere oder deren Mischungen, soweit sie als miteinander verträglich bekannt sind und Trihexenylisocyanurat.

## Claims

1. Process for producing a crosslinked organic polymer by reaction of a polymer with a crosslinking agent, **characterized in that** the crosslinking agent is trihexenyl isocyanurate.

2. Process according to Claim 1, **characterized in that** as polymers thermoplastic polymers selected from the group containing polyvinyl polymers, polyolefins, polystyrenes, polyacrylates, polymethacrylates, polyesters, polyamides, polycarbonates, polyphenylene ethers, polyphenylene sulfides, polyacetals, polyphenylene sulfones, fluoropolymers or mixtures thereof, in so far as they are known to be compatible with one another, are employed.

3. Process according to Claims 1 to 2, **characterized in that** the amount of trihexenyl isocyanurate is from 0.01% to 10% by weight based on the crosslinkable polymer.

4. Process according to Claims 1 to 3, **characterized in that** according to the polymer to be crosslinked the crosslinking is performed either by addition of a suitable peroxide at a temperature of 160°C to 190°C or by electron beam crosslinking at room temperature.

5. Crosslinkable composition, containing a polymer selected from the group of polyvinyl polymers, polyolefins, polystyrenes, polyacrylates, polymethacrylates, polyesters, polyamides, polycarbonates, polyphenylene ethers, polyphenylene sulfides, polyacetals, polyphenylene sulfones, fluoropolymers or mixtures thereof, in so far as they are known to be compatible with one another, and trihexenyl isocyanurate.

## Revendications

1. Procédé de fabrication d'un polymère organique réticulé par mise en réaction d'un polymère avec un agent de réticulation, **caractérisé en ce que** l'agent de réticulation est l'isocyanurate de trihexényle.

2. Procédé selon la revendication 1, **caractérisé en ce que** des polymères thermoplastiques choisis dans le groupe contenant les polymères de polyvinyle, les polyoléfines, les polystyrènes, les polyacrylates, les polyméthacrylates, les polyesters, les polyamides, les polycarbonates, les polyéthers de phénylène, les polysulfures de phénylène, les polyacétals, les polyphénylène-sulfones, les polymères fluorés ou leurs mélanges, dans la mesure où ils sont connus pour être compatibles les uns avec les autres, sont utilisés en tant que polymères.

3. Procédé selon les revendications 1 à 2, **caractérisé en ce que** la quantité d'isocyanurate de trihexényle est de 0,01

à 10 % en poids, par rapport au polymère réticulable.

**4.** Procédé selon les revendications 1 à 3, **caractérisé en ce que** la réticulation est réalisée en fonction du polymère à réticuler soit avec ajout d'un peroxyde approprié à une température de 160 à 190 °C, soit par réticulation par un faisceau d'électrons à température ambiante.

**5.** Composition réticulable, contenant un polymère, choisi dans le groupe constitué par les polymères de polyvinyle, les polyoléfines, les polystyrènes, les polyacrylates, les polyméthacrylates, les polyesters, les polyamides, les polycarbonates, les polyéthers de phénylène, les polysulfures de phénylène, les polyacétals, les polyphénylène-sulfones, les polymères fluorés ou leurs mélanges, dans la mesure où ils sont connus pour être compatibles les uns avec les autres, et de l'isocyanurate de trihexényle.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 10241914
- US 20070208142 A
- US 3108987 A


### In der Beschreibung aufgeführte Nicht-Patentliteratur


- **FANG, QIANG ; JIANG, LUXIA.** *Journal of Applied Polymer Science,* 2001, vol. 81 (5), 1248-1257
- Handbook of Chemistry and Physics. 1972